# EUROPÄISCHE PATENTSCHRIFT

(11) **EP 3 539 604 B1**
(45) Veröffentlichungstag und Bekanntmachung des Hinweises auf die Patenterteilung: **11.08.2021**
(21) Anmeldenummer: 19160749.8
(22) Anmeldetag: 05.03.2019
(51) Int. Cl.: A61M 16/06, A61M 16/20

(54) **SAUERSTOFFTHERAPIEGERÄT**
OXYGEN THERAPY DEVICE
APPAREIL OXYTHÉRAPEUTIQUE

(30) Priorität: 16.03.2018 DE 102018106161; 30.05.2018 DE 102018112903
(43) Veröffentlichungstag der Anmeldung: 18.09.2019
(73) Patentinhaber: Fritz Stephan GmbH Medizintechnik, 56412 Gackenbach (DE)
(72) Erfinder: Schliecker, Jan, 56072 Koblenz (DE); Krekel, Daniel, 65558 Gückingen (DE)
(74) Vertreter: Kaufmann, Sigfrid

(56) Entgegenhaltungen:
- WO-A1-2015/192186
- US-A1- 2011 067 704
- US-A1- 2014 150 789
- US-A1- 2015 083 123

## Beschreibung

Die Erfindung betrifft ein Sauerstofftherapiegerät zur Anwendung in der sog. High-Flow-Sauerstofftherapie.

Die High-Flow-Sauerstofftherapie (HFOT), oder auch als High-Flow-Nasal-Cannula (HFNC) bezeichnet, ist die Applikation eines Sauerstoff-Gasgemisches, im Folgenden Patientengas genannt, bei spontan atmenden Patienten über eine dafür vorgesehene sog. Brille oder Maske. Hierbei wird dem Patienten, abhängig von seinem Gewicht und seiner Anatomie, ein Sauerstoffgemisch in einem Flussbereich von 2 - 60 l/min zugeführt, das somit den inspiratorischen Fluss übersteigt. Dieses Gemisch wird vorher über einen aktiven Befeuchter angewärmt und angefeuchtet. Diese Form der Beatmung wird vor allem in der Neonatologie und Pädiatrie eingesetzt, findet aber zunehmend auch Anwendung in allen Altersstufen.

Durch den hohen Gasfluss während der High-Flow-Sauerstofftherapie wird der anatomische Totraum ausgewaschen, woraus eine unterstützende CO₂ Eliminierung resultiert. Des Weiteren führt die Sauerstofftherapie zu einer Reduktion des Atemwiderstandes.

Aus der WO 2011/029073 A1 ist ein derartiges High-Flow-Sauerstofftherapie-System bekannt, bei welchem zusätzlich eine Düse vorgesehen ist, mittels derer Umgebungsluft dem Patientengas zugemischt wird.

Die EP 2 414 019 B1 und die EP 2 068 992 B1 beschreiben jeweils ein High-Flow-System, das eine Steuereinheit und einen im Gasverteiler der Maske angeordneten Atmungssensor aufweist, wobei die Steuereinheit den Gasfluss auf Basis der mit dem Atmungssensor ermittelten Werte synchron zur Spontanatmung des Patienten steuert.

Die Vorteile der Systeme zur High-Flow-Sauerstofftherapie sind ihre unkomplizierte und schnelle Handhabung. Ein schwerwiegender Nachteil ist, dass die aus dem Stand der Technik bekannten Systeme rein flussgetrieben sind, da eine Rückmeldung über den erreichten trachealen Druck fehlt. So kann deren Anwendung schlimmstenfalls zu einem Barotrauma oder einem Pneumothorax führen.

Bei solchen rein flussgesteuerten Systemen ist es üblich, dass das Krankenhauspersonal eine Highflow-Maske so wählt, dass an den Nasenkanülen eine Leckage von ca. 50% erreicht wird. Damit soll ein zu hoher alveolärer Druck bei Fehlanwendung verhindert werden.

WO 2016/157105 A1 offenbart einen Beatmungsapparat, der ein Nasenteil, einen mit diesem verbundenen Formschlauch sowie ein Ventil umfasst. Der Beatmungsapparat ist ausgelegt, in zwei Konfigurationen dem Patienten Atemgas zuzuführen: mit hohem Druck und mit geringerem Druck, wobei das Umschalten zwischen den beiden Konfigurationen mittels eines Ventils erfolgt, das dem Formschlauch zur Druckreduktion ein künstliches Leck zufügt. Hierzu sind verschiedene Ausgestaltungen eines Überdruckventils gezeigt.

Ein Überdruckventil mit angeschlossener Alarmpfeife, welches per Adapter in einen Beatmungsschlauch einsetzbar ist, wird in US 2010/0282253 A1 offenbart. Dieses Überdruckventil wird mit einer Spiralfeder realisiert. Da hierbei mehrere Komponenten zueinander beweglich angeordnet sind, ist dieses Überdruckventil vergleichsweise komplex aufgebaut, wobei ein Verhaken bzw. Verklemmen der beweglichen Komponenten - und somit ein Versagen des Überdruckventils - nicht auszuschließen ist. Schließlich wird durch den Adapter noch eine zusätzliche "Sollbruchstelle" in den Beatmungskreislauf eingebaut.

CA 2980539 A1 zeigt eine Maske zum Beatmen eines Patienten, das ein Nasenteil und ein Mundstück umfasst. Jedes Teil besitzt einen eigenen Gasanschluss. Beide Teile sind lösbar miteinander verbunden, wobei ein im fluidischen Übergang zwischen Nasenteil und Mundstück angebrachtes Ventil zwei separate Beatmungskreisläufe ermöglicht.

WO 2015/192186, US 2011/0067704, WO2013/148901, US 2014/0150789 offenbaren Nasenkanülen mit Druckregelventilen.

Der Erfindung liegt die Aufgabe zugrunde, die oben genannten Nachteile, wie einen unkontrolliert überhöhten trachealen Druck und die erforderliche Akkuratesse und Erfahrung beim Anlegen des Sauerstofftherapie-Systems, zu vermeiden, wobei ein unkompliziert handzuhabendes Sauerstofftherapiegerät bereitgestellt werden soll, das für den Patienten eine hohe Sicherheit hinsichtlich des bereitgestellten Gasdruckes und eine hohe Zuverlässigkeit hinsichtlich der Gasversorgung bieten soll.

Die Lösung dieser Aufgabe erfolgt gemäß des Patentanspruchs 1; zweckmäßige Ausgestaltungen der Erfindung finden sich in den Unteransprüchen.

Gemäß der Erfindung wird ein Sauerstofftherapiegerät für eine High-Flow-Sauerstofftherapie mit Patientengas für einen Patienten zur Beatmungsunterstützung bereitgestellt, das eine integrierte, zwangsgesteuerte Druckregelungseinrichtung unter Verzicht auf elektronische Komponenten aufweist, wobei zusätzlich speziell gestaltete Formschläuche eine unterbrechungsfreie Versorgung des Patienten mit Patientengas sicherstellen.

Das Sauerstofftherapiegerät umfasst ein Nasenteil zur Anbringung am Patienten, eine Gasverteileinrichtung, wenigstens einen Formschlauch und wenigstens ein mechanisches Druckregelventil.

Das Nasenteil (in der medizinischen Fachsprache auch als Nasenprong bezeichnet) leitet das Patientengas in die Nase des Patienten ein. Es beinhaltet einen Gaskanal für das Patientengas und zwei mit dem Gaskanal in fluidischer Verbindung stehende Nasenkanülen mit je einer Patientengasaustrittsöffnung. Außerdem umfasst das Nasenteil wenigstens eine Gaseintrittsöffnung zur Zuführung von Patientengas in den Gaskanal.

Gemäß einer ersten Variante des Systems weist das Nasenteil außerdem zwei flügelartige Haftelemente mit Haft-Silikon-Gel, im Folgenden Haftfolie genannt, auf. Die beiden Nasenkanülen werden nasal eingeführt und oberhalb der Oberlippe mithilfe von je einer Haftfolie auf jeder Seite des Gesichts befestigt. Somit ist ein Herausrutschen der Nasenkanülen verhindert.

Indem das Nasenteil der Anatomie des Gesichtes folgend leicht gebogen oder, z. B. zwischen den beiden Nasenkanülen, abgewinkelt ausgeführt ist, wird die Zugkrafteinwirkung auf die Haftelemente verringert, wodurch ebenfalls die Belastung auf der Haut verringert ist. Das Nasenteil weist zusätzlich in der Mitte eine leichte Konizität auf, um ein Abknicken zu verhindern.

Gemäß einer zweiten Variante des Systems umfasst das Nasenteil nur eine Gaseintrittsöffnung zur Zuführung von Patientengas, wobei eine Patientengaszuführungsvorrichtung, beispielsweise in Form eines röhrenartigen Elements, in ihrem ersten Endbereich mit dem Formschlauch verbindbar ist. In diesem Bereich weist die Patientengaszuführungsvorrichtung wenigstens eine Haftfolie auf, mittels derer sie auf der Stirn des Patienten positioniert wird. Dadurch würde die sensible Haut der Wange nicht zusätzlich beansprucht und gereizt werden, wodurch ein deutlich größerer Komfort für den Patienten entsteht.

Die beiden Nasenkanülen, die an dem zweiten Endbereich der Patientengaszuführungsvorrichtung angeordnet sind, können lösbar mit derselben verbunden sein. Hierdurch kann in vorteilhafter Weise eine Patientengaszuführungsvorrichtung in einer einheitlichen Größe verwendet werden, wobei nur die Nasenkanülen patientenspezifisch auszutauschen sind. So ist möglich, dass nur durch das Tauschen des Nasenteils unterschiedliche Größen an Patiententypen adaptiert werden können.

Zudem ist es möglich, dass das Nasenteil als Einzelteil ausgelegt wird, sodass sich dieses an die Gasverteilereinrichtung adaptieren, stecken, clipsen oder aufschieben lässt.

Die Patientengaszuführungsvorrichtung wird als zentrale Zuführung oberhalb des Nasenrückens ausgeführt, wobei diese einen ähnlichen großen Querschnitt aufweist, wie der der Zuleitung (Schlauchsystem). So kann bis hin zu den Nasenkanülen, deren Durchmesser gegenüber dem Durchmesser des Gaskanals des Nasenteils verringert ist, ein starker Druckverlust im System verhindert werden.

Das Nasenteil gemäß dieser Variante kann im Nasenbereich so ausgelegt sein, dass die Form der Nasenkontur nachempfunden ist, sodass sich das Nasenteil der Nase anschmiegt. Dieser Bereich kann zudem etwas weicher ausgeführt sein. Dadurch ist das Gesamtsystem deutlich besser geführt, sodass einem fehlerhaften Positionieren oder Verrutschen vorgebeugt werden kann, wobei außerdem aufgrund des weichen Bereichs eine Reizung oder Beschädigung im Bereich der Nase des Patienten vermieden wird.

Beide Varianten weisen Nasenkanülen auf, die so zum Patienten hin gewinkelt sind, dass diese sich dem Nasenvorhof anpassen, wodurch eine Beschädigung oder Reizung der Nasenwand vermieden wird. Außerdem weisen die Nasenkanülen an ihrem Endbereich mit der Patientengasaustrittsöffnung eine Abschrägung bzw. Fase auf, damit zum einen die Kanülen nicht gegen die Naseninnenwand drücken und zum anderen der Gasfluss in die Nase hinein wenig beeinträchtigt wird.

Die Gasverteileinrichtung dient dem Anschluss des Sauerstofftherapiegeräts an eine Patientengasquelle. Hierzu weist die Gasverteileinrichtung einen Adapteranschluss für einen grundsätzlich bekannten Gaszuführungsschlauch von einer Patientengasquelle auf. Diesem Adapteranschluss gegenüberliegend weist die Gasverteileinrichtung eine oder zwei Anschlussbuchsen auf, in die jeweils ein Formschlauch des Sauerstofftherapiegeräts einbringbar ist. Jede Anschlussbuchse definiert somit eine Gasaustrittsöffnung der Gasverteileinrichtung.

Die Anschlussbuchsen weisen innenliegend und von der Formschlauch-Einführungsöffnung beabstandet eine Arretiereinrichtung, z. B. in Form eines umlaufenden Wulstes oder eines Absatzes auf, sodass der Formschlauch nur maximal bis zu der Arretiereinrichtung in die Anschlussbuchse der Gasverteileinrichtung einschiebbar ist. Um eine gasdichte Verbindung zwischen Formschlauch und Anschlussbuchse zu erhalten, entspricht der Innenquerschnitt der Anschlussbuchse in Form und Abmessung im Wesentlichen der Außenquerschnittsform des Formschlauches.

Der Formschlauch weist eine Gaseintrittsöffnung und eine Gasaustrittsöffnung auf, wobei der Formschlauch mit seiner Gasaustrittsöffnung in eine Gaseintrittsöffnung des Nasenteils mündet. Der diesem Endbereich gegenüberliegende Endbereich des Formschlauches, nämlich der Endbereich mit der Gaseintrittsöffnung, ist bzw. wird in eine bzw. die Anschlussbuchse der Gasverteileinrichtung eingeführt und ist somit an eine Gasaustrittsöffnung der Gasverteileinrichtung angeschlossen. Um ein versehentliches Lösen des Formschlauches von dem Nasenteil und/oder der Gasverteileinrichtung zu verhindern, kann der Formschlauch zusätzlich jeweils mit einem Kleber, z. B. einem Silikon-Kleber, fixiert sein.

Der Formschlauch ist in grundsätzlich bekannter Weise zylinder- bzw. rohrförmig ausgebildet. Im Querschnitt kann der Formschlauch eine runde oder elliptische Außengeometrie aufweisen. Die geometrische Form des Querschnitts der Innenwandung, d. h. die (innere) Querschnittsform des Gaskanals im Formschlauch, weicht erfindungsgemäß von der Form einer Ellipse, insbesondere also auch von der Form eines Kreises, ab. Erfindungsgemäß weist der Formschlauch eine Querschnittsform seines Gaskanals auf, die stern-, blumen-, kreissektor- oder polygonförmig ist.

Hierbei kann auch vorgesehen sein, dass in Längsrichtung des Formschlauches zwei oder mehr Gaskanäle ausgebildet sind, die im Wesentlichen parallel zueinander verlaufen. Beispielsweise kann der Formschlauich vier Gaskanäle aufweisen, die jeweils eine kreissektorförmige Querschnittsform aufweisen. Die Gaskanäle können entlang der gesamten Länge des Formschlauches strikt voneinander getrennt verlaufen, sie können aber auch an einer oder mehreren Positionen fluidische Verbindungen untereinander aufweisen.

Durch die spezielle, von einer Ellipsenform abweichende innere Kontur des Formschlauches, d. h. Querschnittsform des Gaskanals im Formschlauch, wird im Falle eines Knicks ein Restquerschnitt offen gehalten. Dieser garantiert einen minimalen Restfluss des Patientengases. Ebenso erschwert die Kontur ein unbeabsichtigtes Knicken des Formschlauches.

Das Druckregelventil umfasst mindestens eine Ventilklappe, wobei dessen Funktionsweise rein mechanisch erfolgt, d. h. es weist keine elektrischen Komponenten auf. Das Druckregelventil ist eingerichtet, sowohl eine negative Druckdifferenz des Patientengasdruckes in dem Sauerstofftherapiegerät zum Atmosphärendruck, als auch eine positive Druckdifferenz zum Atmosphärendruck auszugleichen. Das Druckregelventil bzw. die mindestens eine Ventilklappe ist ausgelegt, oberhalb eines vorgegebenen Betrags der Druckdifferenz zu öffnen, wobei ein Öffnungsgrad der Ventilklappe proportional der Druckdifferenz ist.

Vorzugsweise ist das wenigstens eine Druckregelventil integraler Bestandteil des Nasenteils oder der Gasverteileinrichtung, wobei das Druckregelventil monolithisch mit dem Nasenteil bzw. der Gasverteileinrichtung ausgebildet ist.

Somit schützt das in dem Sauerstofftherapiegerät integrierte Druckregelventil in vorteilhafter Weise den Patienten. Denn durch ein Zusammenspiel von eingestelltem Gasfluss, den Nasenkanülen und der Nasenform des Patienten werden verschiedene Faktoren wie z. B. die Leckage am Übergang Kanüle - Nase beeinflusst. Dadurch können, je nach Situation, unterschiedliche Drücke in den Atemwegen des Patienten anliegen. Bei einem zu hohem Druck öffnen sich die Ventilklappen und bewahren den Patienten vor einem zu hohen trachealen Druck, sodass ein Barotrauma oder ein Pneumothorax zuverlässig vermieden wird.

Das Druckregelventil kann in Form eines Zylinders oder eines Quaders ausgebildet sein, durch den das Patientengas strömt, wobei im Falle eines Zylinders die Gasströmrichtung bevorzugt quer zur Zylinderlängsachse verläuft. Wenigstens eine Seitenwand des Quaders oder eine Deckfläche des Zylinders kann eine Silikonmembran sein, in die erfindungsgemäß mindestens zwei sich kreuzende Schlitze eingebracht sind. Hierdurch sind erfindungsgemäß wenigstens vier bewegliche Ventilklappen gebildet, die sich - mit einer dem Material inhärenten Federkraft behaftet - frei nach außen und nach innen bewegen können.

Die Konturen ermöglichen hier ein definiertes Öffnungsverhalten der Ventilklappen abhängig von der Druckdifferenz des innenanliegenden Systemdrucks zum Atmosphärendruck. Bei Anliegen einer Druckdifferenz zwischen Gaskanal und Außenatmosphäre kann somit ein Druckausgleich erfolgen.

Um die empfindliche Silikonmembran vor mechanischer Einwirkung zu schützen, kann außen auf dem Druckregelventil oberhalb der Silikonmembran eine Schutzkappe angebracht sein. Diese Schutzkappe kann die Ventilklappen teilweise oder vollständig überdecken, wobei hierbei noch, beispielsweise durch seitlich in die Schutzkappe eingebrachte Schlitze, ein Gasaustausch ermöglicht ist.

Auch kann vorgesehen sein, dass die Schutzkappe das gesamte Druckregelventil teilweise oder vollständig umschließt. Da insbesondere vorgesehen ist, dass das Druckregelventil monolithisch in die entsprechende Komponente des Sauerstofftherapiegeräts, nämlich das Nasenteil oder die Gasverteileinrichtung, intergiert ist, kann auch vorgesehen sein, dass besagte Komponente vollständig aus dem Material des Druckregelventils bzw. der Ventilklappen besteht. Somit bietet die Schutzkappe, die aus einem anderen Material bestehen kann, eine zusätzliche Stabilisierung des weichen Silikon-Ventilgehäuses.

Die Erfindung kann weiter derart ausgebildet sein, dass die Gasverteileinrichtung ein oder zwei Druckregelventile aufweist. Vorzugsweise umfasst die Gasverteileinrichtung zwei Druckregelventile, die - sich gegenüberliegend - zwischen dem Adapteranschluss und den ein oder zwei Anschlussbuchsen angeordnet sind.

Alternativ kann vorgesehen sein, dass das Nasenteil ein oder zwei Druckregelventile aufweist, die vorzugsweise im Gaskanal zwischen Gaseintrittsöffnung des Nasenteils und Nasenkanüle angeordnet sind. Das Druckregelventil im Nasenteil ermöglicht in vorteilhafter Weise, neben dem Schutz vor einem zu hohen Patientendruck, außerdem eine unbehinderte Spontanatmung des Patienten, sodass er nicht gegen den Widerstand des ganzen Atemsystems bzw. Schlauchsystems atmen muss.

Zudem kann es sein, dass mindestens ein Druckregelventil in dem Stirn-Haft-Pad integriert ist oder dort befestigt werden kann.

Weiterhin kann vorgesehen sein, dass die Nasenkanülen an ihrer Patientengasaustrittsöffnung eine Leckageeinrichtung aufweisen, d. h., es existiert keine gasdichte Verbindung zwischen Nase des Patienten und Nasenteil. Diese Nasenteilvariante mit definierter Leckage an der Nasenkanüle dient zum genaueren Bestimmen des Patientendrucks. Durch die bekannte Leckage und den vom Gerät abgegebenen Gasfluss und Druck kann so, beispielsweise mittels eines Mess- und Regelbauteils, näherungsweise der Patientendruck bestimmt werden.

Weist das Nasenteil zusätzlich ein oder zwei Druckregelventile auf, so kann außerdem durch die Kombination des Ventils und der definierten Leckage über den Druckverlauf des Ventils ebenfalls der Patientendruck näherungsweise bestimmt werden.

Die Leckageeinrichtung kann in Form eines konzentrisch zur Nasenkanülenlängsachse angeordneten Ringes oder Hohlzylinders ausgebildet sein, der mittels Abstandshaltern außen an der Wandung der Nasenkanüle im Bereich der Patientengasaustrittsöffnung befestigt ist.

Gemäß einer Ausgestaltung des Sauerstofftherapiegeräts umfasst es nur einen Formschlauch. Beim Anlegen des Sauerstofftherapiegeräts wird dieser Formschlauch oberhalb des Nasenrückens verlaufend über den Kopf des Patienten gelegt.

Gemäß einer alternativen Ausgestaltung des Sauerstofftherapiegeräts umfasst es zwei Formschläuche, die jeweils von der Gasverteileinrichtung zu je einer Gaseintrittsöffnung des Nasenteils geführt sind. Die beiden Formschläuche werden beim Anlegen des Sauerstofftherapiegeräts über die Ohren gelegt (ähnlich einem Brillenbügel). Gemäß dieser Ausgestaltung weist das Sauerstofftherapiegerät zusätzlich eine Schlauchführung auf, mittels derer die beiden Formschläuche aneinander fixiert sind. Die Schlauchführung ist hierbei entlang (der Längsachsen) der Formschläuche verschiebbar ausgeführt. Somit kann nach Anlegen des Sauerstofftherapiegeräts durch Verschieben der Schlauchführung hin zum Kopf des Patienten ein "Festzurren" der Formschläuche und damit des Therapiegerätes am Kopf des Patienten erfolgen.

Gemäß einer Ausgestaltung ist der Innenquerschnitt im Formschlauch entlang der Formschlauchlängsachse um dieselbe gedreht, sodass der Gaskanal helixförmig ausgebildet ist. Im Falle der Formschlauch mehrere Gaskanäle beinhaltet, können diese jeweils um die eigene Gaskanallängsachse entlang derselben gedreht sein. Alternativ oder zusätzlich können die Gaskanäle im Sinne einer Mehrfachhelix entlang der Formschlauchlängsachse umeinander geführt, d. h. gewickelt, sein.

Durch dieses Verdrehen der Innenquerschnittsform bleibt im Falle eines Knicks im Schlauch ein kleiner Restquerschnitt für den Gasfluss geöffnet, wobei durch das Verdrehen entlang der Längsachse der Knickwiderstand erheblich vergrößert wird.

Zusätzlich oder alternativ kann in den Formschlauch ein offenporiger Schaum eingebracht sein, der den Gaskanal vollständig ausfüllt. Der offenporige Schaum erlaubt einen ungestörten Gasfluss, wobei ein Zusammendrücken oder Knicken des Formschlauches, und damit eine Unterbrechung des Gasflusses, sicher verhindert wird, sodass stets die Versorgung des Patienten mit Patientengas gewährleistet ist.

Die Erfindung wird nachfolgend anhand von Ausführungsbeispielen mit Bezug auf die Figuren veranschaulicht, wobei gleiche oder ähnliche Merkmale mit gleichen Bezugszeichen versehen sind. Hierzu zeigen in schematischer Darstellung die
- Figur 1:: das Sauerstofftherapiegerät in perspektivischer Sicht;
- Figur 2:: eine Ausgestaltung des Nasenteils in perspektivischer Sicht;
- Figur 3:: eine weitere Ausgestaltung des Nasenteils in perspektivischer Sicht;
- Figur 4: eine Ausgestaltung der Gasverteileinrichtung in perspektivischer Sicht und im perspektivischen Längsschnitt;
- Figur 5: eine Ausgestaltung der Gasverteileinrichtung mit Schutzkappe in perspektivischer Sicht;
- Figur 6: Varianten des Formschlauches im Querschnitt;
- Figur 7: eine dritte Ausgestaltung des Nasenteils in perspektivischer Sicht; und
- Figur 8: Detail einer vierten Ausgestaltung des Nasenteils in perspektivischer Sicht.

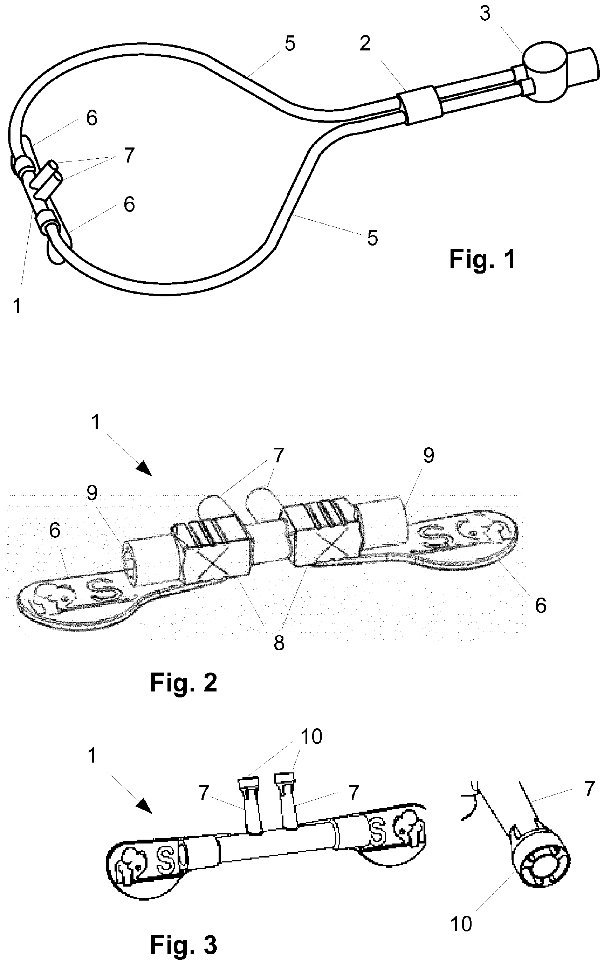

Die Figur 1 zeigt das Sauerstofftherapiegerät mit dem Nasenteil 1, zwei Formschläuchen 5 und der Gasverteileinrichtung 3. Das Nasenteil 1 umfasst die beiden Nasenkanülen 7 sowie die Haftfolien 6 zur Fixierung des Nasenteils auf der Haut des Patienten. Die beiden Formschläuche 5 werden durch die verschiebbare Schlauchführung 2 zusammen gehalten.

Das Nasenteil 1 gemäß Figur 2 umfasst die beiden Druckregelventile 8, die im Gaskanal des Nasenteils 1 zwischen Gaseintrittsöffnung 9 und Nasenkanüle 7 angeordnet sind. Die Haftfolie 6 ist in diesem Beispiel eine Polyurethane Folie mit Haft-Silicone-Gel.

Die Nasenkanülen 7 des Nasenteils 1 gemäß Figur 3 weisen jeweils die Leckageeinrichtung 10 auf, die in Form eines konzentrisch um die Nasenkanüle 7 angeordneten Ringes ausgeführt ist, wie in der Detailansicht auf der rechten Seite von Fig. 3 ersichtlich.

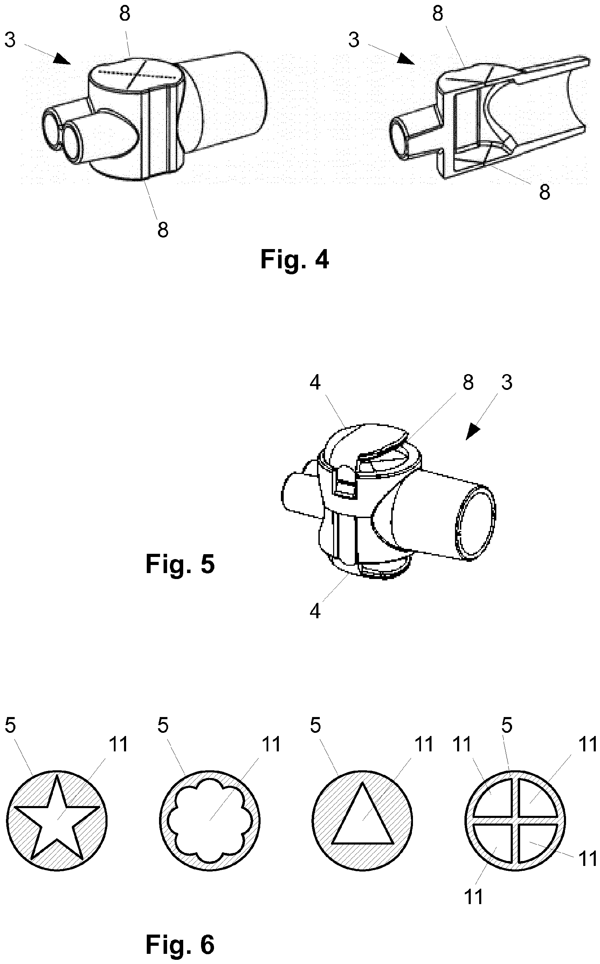

Figur 4 zeigt eine Ausgestaltung der Gasverteileinrichtung 3 mit zwei Druckregelventilen 8, die hier in Form einer Silikonfolie mit zwei x-förmig eingebrachten Schlitzen ausgeführt sind.

In Figur 5 ist eine zu der Ausführung gemäß Fig. 4 baugleiche Gasverteileinrichtung 3 mit zusätzlicher Schutzkappe 4 dargestellt. In diesem Beispiel lässt die Schutzkappe 4 seitlich am Druckregelventil 8 einen Spalt frei, sodass ein ungehinderter Druckausgleich ermöglicht ist.

Verschiedene Ausführungsformen des Querschnitts des Gaskanals 11 im Formschlauch 5 sind in Fig. 6 zu sehen. Insbesondere zeigt die Variante ganz rechts einen Formschlauch 5 mit vier Gaskanälen 11, die jeweils einen kreissektorförmigen Querschnitt aufweisen.

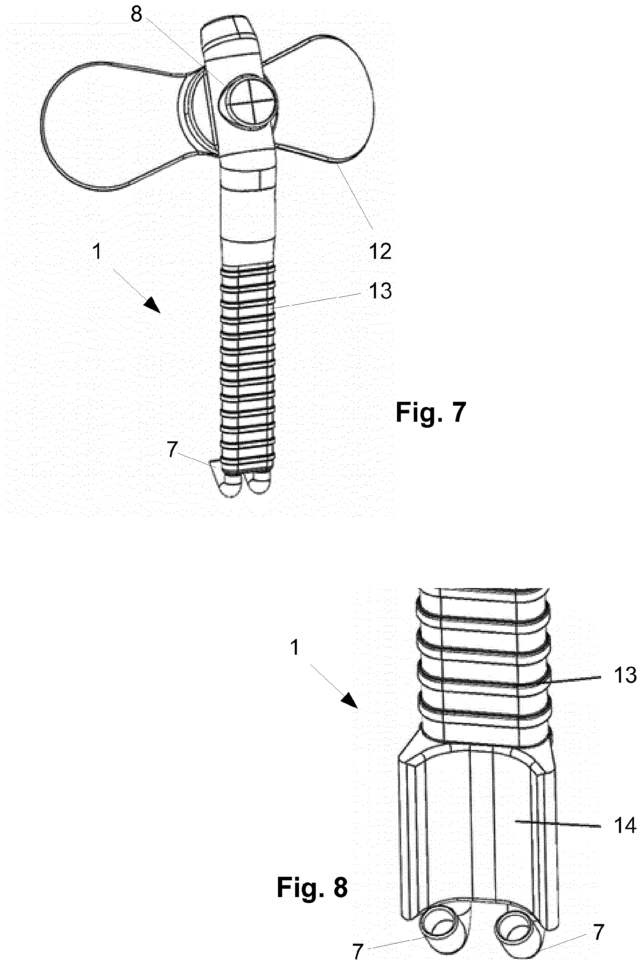

Die Fig. 7 zeigt eine Variante des Nasenteils 1 mit der Patientengaszuführungsvorrichtung 13 und dem Druckregelventil 8, welches sich im Bereich der Stirnauflage 12 mit Haft-Silikon-Gel befindet.

Die Variante des Nasenteils 1 gemäß Fig. 8 weist die Nasenrückenkontur 14 auf, an welcher die Nasenkanülen 7 lösbar befestigt sind.

### Liste der verwendeten Bezugszeichen

- 1: Nasenteil
- 2: Schlauchführung
- 3: Gasverteileinrichtung
- 4: Schutzkappe
- 5: Formschlauch
- 6: Haftfolie
- 7: Nasenkanüle
- 8: Druckregelventil
- 9: Gaseintrittsöffnung des Nasenteils
- 10: Leckageeinrichtung
- 11: Gaskanal im Formschlauch
- 12: Stirnauflage mit Haft-Silikon-Gel
- 13: Patientengaszuführungsvorrichtung
- 14: Formkontur zur Führung an der Nase

## Patentansprüche

1. Sauerstofftherapiegerät für eine High-Flow-Sauerstofftherapie, aufweisend:
- ein Nasenteil (1), umfassend einen Gaskanal für ein Patientengas und zwei mit dem Gaskanal in fluidischer Verbindung stehende Nasenkanülen (7) mit je einer Patientengasaustrittsöffnung,
- wenigstens ein Druckregelventil (8), das eine Silikonmembran ist, in welche mindestens zwei sich kreuzende Schlitze eingebracht sind, wodurch wenigstens vier, nach außen und nach innen bewegliche Ventilklappen gebildet sind, und
- wenigstens einen Formschlauch (5), aufweisend mindestens einen Gaskanal (11), der in eine Gaseintrittsöffnung und eine Gasaustrittsöffnung des Formschlauches (5) mündet, wobei der Formschlauch (5) mit seiner Gasaustrittsöffnung in eine Gaseintrittsöffnung (9) des Nasenteils (1) mündet, und
- das Sauerstofftherapiegerät eine Gasverteileinrichtung (3) zum Anschluss an eine Patientengasquelle aufweist, wobei der wenigstens eine Formschlauch (5) mit seiner Gaseintrittsöffnung in eine Gasaustrittsöffnung der Gasverteileinrichtung (3) mündet,
- der Formschlauch (5) eine Querschnittsform seines mindestens einen Gaskanals (11) aufweist, die stern-, blumen-, kreissektor- oder polygonförmig ist, und
- die wenigstens vier, nach außen und nach innen beweglichen Ventilklappen des wenigstens einen Druckregelventils (8) sich bei Vorliegen eines Differenzdrucks zwischen Atmosphärendruck und Gasdruck in der Gasverteileinrichtung (3) und/oder dem Nasenteil (1) mit einer dem Ventilklappen-Material inhärenten Federkraft behaftet proportional dem Differenzdruck öffnen.

2. Sauerstofftherapiegerät nach Anspruch 1, **dadurch gekennzeichnet, dass** das Nasenteil (1) wenigstens ein mit dem Gaskanal in fluidischer Verbindung stehendes Druckregelventil (8) aufweist.

3. Sauerstofftherapiegerät nach Anspruch 1, **dadurch gekennzeichnet, dass** das wenigstens eine Druckregelventil (8) Bestandteil der Gasverteileinrichtung (3) ist.

4. Sauerstofftherapiegerät nach einem der vorstehenden Ansprüche, **dadurch gekennzeichnet, dass** die Nasenkanülen (7) an ihrer Patientengasaustrittsöffnung eine Leckageeinrichtung (10) aufweisen.

5. Sauerstofftherapiegerät nach Anspruch 4, **dadurch gekennzeichnet, dass** die Leckageeinrichtung (10) einen konzentrisch zur Nasenkanülenlängsachse angeordneten Ring umfasst, der mittels Abstandshaltern außen an der Wandung der Nasenkanüle (7) befestigt ist.

6. Sauerstofftherapiegerät nach einem der vorstehenden Ansprüche, **dadurch gekennzeichnet, dass** das wenigstens eine Druckregelventil (8) monolithisch in das Nasenteil (1) oder die Gasverteileinrichtung (3) intergiert ist.

7. Sauerstofftherapiegerät nach Anspruch 6, **dadurch gekennzeichnet, dass** das Druckregelventil (8) von außen von einer Schutzkappe zumindest teilweise abgedeckt ist.

8. Sauerstofftherapiegerät nach einem der vorstehenden Ansprüche, **dadurch gekennzeichnet, dass** es zwei Formschläuche (5) aufweist, die jeweils mit ihrer Gasaustrittsöffnung mit dem Gaskanal des Nasenteils (1) und mit ihrer Gaseintrittsöffnung mit der Gasverteileinrichtung (3) verbunden sind, wobei die beiden Formschläuche (5) mittels einer entlang der Formschläuche (5) verschiebbaren Schlauchführung (2) aneinander fixiert sind.

9. Sauerstofftherapiegerät nach einem der vorstehenden Ansprüche, **dadurch gekennzeichnet, dass** der Formschlauch (5) vier Gaskanäle (11) beinhaltet, die jeweils eine kreissektorförmige Querschnittsform aufweisen.

10. Sauerstofftherapiegerät nach einem der vorstehenden Ansprüche, **dadurch gekennzeichnet, dass** der Gaskanal (11) im Formschlauch (5) helixförmig ausgebildet ist, indem die Querschnittsform des Gaskanals (11) entlang der Längsachse des Formschlauches (5) gedreht ist.

## Claims

1. Oxytherapy instrument for high flow oxygen therapy, presenting:
- a nasal part (1) comprising a gas channel for a patient's gas and two nasal cannulas (7) being in fluid communication with the gas channel, each with a patient gas outlet opening,
- at least one pressure regulating valve (8) which is a silicone membrane into which at least two intersecting slots are introduced, by which at least four movable valve flaps are formed outwardly and inwardly, and
- at least one preformed tube (5), having at least one gas channel (11) which opens into a gas inlet opening and a gas outlet opening of the preformed tube (5), in which the preformed tube (5) opens with its gas outlet opening into a gas inlet opening (9) of the nasal part (1), and
- the oxytherapy instrument has a gas distribution device (3) intended to be connected to a patient gas source, in which the at least one preformed tube (5) opens with its gas inlet opening into a gas outlet opening of the gas distribution device (3),
- the preformed tube (5) has a cross-sectional shape of its at least one gas channel (11) which is in the shape of a star, flower, arc of a circle or polygon, and
- the at least four valve flaps movable outwardly and inwardly of the at least one pressure regulating valve (8) open in proportion to the differential pressure in the presence of a differential pressure between the pressure atmospheric and gas pressure in the gas distribution apparatus (3) and / or the nasal part (1) with a spring force inherent in the valve plug material.

2. Oxytherapy instrument according to claim 1, **characterized in that** the nasal part (1) has at least one pressure regulating valve (8) in fluid communication with the gas channel.

3. Oxytherapeutic instrument according to claim 1, **characterized in that** at least one pressure regulating valve (8) is a constituent part of the oxytherapeutic instrument (3).

4. Oxytherapy instrument according to any one of the preceding claims, **characterized in that** the nasal cannulas (7) have a leakage device (10) at their patient gas outlet opening.

5. Oxytherapeutic instrument according to claim 4, **characterized in that** the leakage apparatus (10) comprises a ring arranged concentrically with respect to the longitudinal axis of the nasal cannulas, which is fixed to the outside of the wall of the nasal cannula. (7) by means of spacers.

6. Oxytherapy instrument according to any one of the preceding claims, **characterized in that** the at least one pressure regulating valve (8) is integrated monolithically in the nasal part (1) or the gas distribution device. (3).

7. Oxytherapy instrument according to claim 6, **characterized in that** the pressure regulating valve (8) is at least partially covered from the outside by a protective cap.

8. Oxytherapy instrument according to any one of the preceding claims, **characterized in that** it has two preformed tubes (5), which are respectively connected by their gas outlet opening to the gas channel of the nasal part (1) and through their gas inlet opening to the gas distribution device (3), in which the two preformed tubes (5) are fixed to each other by means of a tube guide (2) which can be moved along the preformed tubes (5).

9. Oxytherapy instrument according to any one of the preceding claims, **characterized in that** the preformed tube (5) contains four gas channels (11) which respectively have a cross-sectional shape in the form of an arc of a circle.

10. Oxytherapy instrument according to any one of the preceding claims, **characterized in that** the gas channel (11) in the preformed tube (5) is made in a helical shape, **in that** the cross sectional shape of the gas channel (11) is rotated along the longitudinal axis of the preformed tube (5).

## Revendications

1. Instrument oxythérapeutique pour une oxygénothérapie à haut débit, présentant :
- une partie nasale (1) comprenant un canal de gaz pour un gaz de patient et deux canules nasales (7) se trouvant en communication fluidique avec le canal de gaz, chacune avec une ouverture de sortie de gaz de patient,
- au moins une soupape de régulation de pression (8) qui est une membrane de silicone dans laquelle au moins deux fentes se croisant sont introduites, par laquelle au moins quatre clapets de soupape mobiles sont formés vers l'extérieur et vers l'intérieur, et
- au moins un tube préformé (5), présentant au moins un canal de gaz (11) qui débouche dans une ouverture d'entrée de gaz et une ouverture de sortie de gaz du tube préformé (5), dans lequel le tube préformé (5) débouche avec son ouverture de sortie de gaz dans une ouverture d'entrée de gaz (9) de la partie nasale (1), et
- l'instrument oxythérapeutique présente un appareil de répartition de gaz (3) destiné à être raccordé à une source de gaz de patient, dans lequel l'au moins un tube préformé (5) débouche avec son ouverture d'entrée de gaz dans une ouverture de sortie de gaz de l'appareil de répartition de gaz (3),
- le tube préformé (5) présente une forme de section transversale de son au moins un canal de gaz (11) qui est en forme d'étoile, de fleur, d'arc de cercle ou de polygone, et
- les au moins quatre clapets de soupape mobiles vers l'extérieur et vers l'intérieur de l'au moins une soupape de régulation de pression (8) s'ouvrent proportionnellement à la pression différentielle en présence d'une pression différentielle entre la pression atmosphérique et la pression de gaz dans l'appareil de répartition de gaz (3) et/ou la partie nasale (1) avec une force de ressort inhérente au matériau de clapet de soupape.

2. Instrument oxythérapeutique selon la revendication 1, **caractérisé en ce que** la partie nasale (1) présente au moins une soupape de régulation de pression (8) en communication fluidique avec le canal de gaz.

3. Instrument oxythérapeutique selon la revendication 1, **caractérisé en ce que** l'au moins une vanne de régulation de pression (8) est une partie constituante de l'instrument oxythérapeutique (3).

4. Instrument oxythérapeutique selon l'une quelconque des revendications précédentes, **caractérisé en ce que** les canules nasales (7) présentent un appareil de fuite (10) au niveau de leur ouverture de sortie de gaz de patient.

5. Instrument oxythérapeutique selon la revendication 4, **caractérisé en ce que** le appareil de fuite (10) comprend un anneau disposé concentriquement par rapport à l'axe longitudinal de canules nasales, qui est fixé à l'extérieur de la paroi de la canule nasale (7) au moyen d'écarteurs.

6. Instrument oxythérapeutique selon l'une quelconque des revendications précédentes, **caractérisé en ce que** l'au moins une soupape de régulation de pression (8) est intégrée de façon monolithique dans la partie nasale (1) ou l'appareil de répartition de gaz (3).

7. Instrument oxythérapeutique selon la revendication 6, **caractérisé en ce que** la soupape de régulation de pression (8) est au moins partiellement recouverte de l'extérieur par un capuchon de protection.

8. Instrument oxythérapeutique selon l'une quelconque des revendications précédentes, **caractérisé en ce qu'**il présente deux tubes préformés (5), qui sont respectivement connectés par leur ouverture de sortie de gaz au canal de gaz de la partie nasale (1) et par leur ouverture d'entrée de gaz à l'appareil de répartition de gaz (3), dans lequel les deux tubes préformés (5) sont fixés l'un à l'autre au moyen d'un guide de tube (2) qui peut être déplacé le long des tubes préformés (5).

9. Instrument oxythérapeutique selon l'une quelconque des revendications précédentes, **caractérisé en ce que** le tube préformé (5) contient quatre canaux de gaz (11) qui présentent respectivement une forme de section transversale en forme d'arc de cercle.

10. Instrument oxythérapeutique selon l'une quelconque des revendications précédentes, **caractérisé en ce que** le canal de gaz (11) dans le tube préformé (5) est réalisé en forme hélicoïdale, **en ce que** la forme de section transversale du canal de gaz (11) est tournée le long de l'axe longitudinal du tube préformé (5).
